# EUROPEAN PATENT APPLICATION

(11) **EP 1 284 135 A2**
(43) Date of publication of application: **19.02.2003**
(21) Application number: 02255693.0
(22) Date of filing: 15.08.2002
(51) Int. Cl.: A61K 7/50, C11D 11/02

(54) **Mild cleansing composition having stable foam**

(30) Priority: 16.08.2001 US 931703; 17.09.2001 US 954335
(71) Applicant: Johnson & Johnson Consumer Companies, Inc., Skillman, New Jersey 08558-9418 (US)
(72) Inventor: Booker, Gregory, Philadelphia, PA 19130 (US); Librizzi, Joseph J., Neshanic, NJ 08853 (US)
(74) Representative: Mercer, Christopher Paul

(57) **Abstract**

A mild cleansing composition that produces a stable foam containing at least one glycoside nonionic surfactant; at least one betaine surfactant; and at least one amphoteric surfactant, is disclosed. The composition is substantially free of anionic surfactant.

## Description

### Cross-Reference to Related Application

This Application is a continuation-in-part application of United States Application Number 09/931,703 filed on 16 August 2001, which is incorporated herein by reference in its entirety.

### Background of the Invention

### 1. Field of the Invention

The present invention relates to a skin and hair cleansing composition capable of producing stable foam and having a low degree of ocular and skin irritation. More specifically, this invention is related to such compositions having a mixture of a betaine surfactant, an amphoteric surfactant, and a nonionic surfactant. The compositions are substantially free of anionic surfactants.

### 2. Description of the Prior Art

It is well known that many surfactants used in shampoos and cleansing compositions are irritating to the eyes, which is of particular concern in shampoos and cleansing compositions used on infants and children. As a result, several less irritating surfactants have been developed.

Foam is a critical property of cleansing compositions, especially personal care products such as shampoos, body washes, hand cleansers, and facial washes. Foam quality and quantity is an important cue to the consumer that the product is an efficacious, quality product. Foam also serves a functional property in that it helps emulsify dirt and oil from the skin and hair and carries the emulsified dirt away.

Surfactants, especially anionic surfactants, are the primary foaming ingredients found in cleansing compositions today. Anionic surfactants, such as sulfates, sulfonates, isethionates, sulfosuccinates, and taurates, are known for providing large quantities of very stable foam. However, anionic surfactants are not without their drawbacks. For example, anionic surfactants may strip skin and hair of their natural oils, which thereby damages hair and leaves skin dry and compromised. Anionic surfactants are also known as irritants to the skin and eyes. For these reasons, compositions containing anionic surfactants may be unsuitable for baby products or for products intended for people with damaged or sensitive skin.

Additionally, anionic surfactants are often prone to hydrolysis, which renders them unsuitable for use in a low pH environment. Furthermore, anionic surfactants may be incompatible with cationic ingredients, which thereby renders them unsuitable for a number of applications.

Alternative surfactants that have been used to minimize the deleterious effects of anionic surfactants include both amphoteric and nonionic surfactants. However, although such surfactants do not possess the above negative properties associated with anionic surfactants, they disadvantageously tend to generate poor, unstable foam relative to that generated by anionic surfactants. This is one reason as to why they are not widely used as primary foaming agents. But see Patent Cooperation Treaty Patent Application No. WO 97/47171 foaming systems consisting of an alkylpolyglucoside nonionic surfactant and an amphoteric surfactant ). Although such amphoteric-non ionic systems are capable of generating foam, that foam is not stable, i.e. long-lasting.

Therefore, there is a need for a cleansing composition based on amphoteric and nonionic surfactants that provides a large quantity of stable, dense foam, but which does not possess the known deleterious effects associated with anionic surfactants.

### Summary of the Invention

The present invention provides a mild cleansing composition capable of producing a stable foam comprising, consisting essentially of, and/or consisting of: at least one glycoside nonionic surfactant; at least one betaine surfactant; and at least one amphoteric surfactant. wherein the composition is substantially free of anionic surfactant.

We have unexpectedly found that the cleansing compositions of the present invention are not only effective in providing a superior stable foam, but do so with a low degree of skin and ocular irritation.

### Detailed Description of Preferred Embodiments

The first component of the compositions of the present invention is at least one glycoside nonionic surfactant. Glycosides are nonionic surfactants that have a hydrophobic group containing about 6 to about 30 carbon atoms and a polysaccharide (polyglycoside) hydrophilic group containing about 1 to about 10 saccharide units. Suitable glycosides include, but are not limited to, coco glucoside, octyl glucoside, decyl glucoside, lauryl glucoside, and mixtures thereof. The amount of glycoside in the compositions of the present invention may range from about 0.1% to about 20%, e.g. from about 0.5% to about 15% or from about 1.0% to about 10% by weight, based on the total weight of the composition.

The second component of the compositions of the present invention is at least one betaine surfactant. As used herein, betaines are typically either cationic at low pH, i.e., at about 4 or less, or isoelectric at intermediate pH, e.g. at about 7. Suitable betaine surfactants include, but are not limited to, alkyl betaines such as decyl betaine, alkyl sultaine such as lauryl hydroxysultane, amidoalkyl betaines such as cocamidopropylbetaine, amidoalkylsultaine such as oleamidopropylhydroxysultaine, and mixtures thereof, wherein the alkyl group contains from about C₈ to about C₂₂ carbon atoms. The amount of betaine surfactant in the compositions of the present invention may range from about 0.1% to about 20%, e.g. from about 0.5% to about 15% or from about 1.0% to about 10% by weight, based on the total weight of the composition.

The third component of the compositions of the present invention is at least one amphoteric surfactant. which may exist in three different forms depending upon pH: cationic at low pH, i.e. at about 4 or less, zwitterionic at intermediate pH, i.e. at about 7, and anionic at high pH, i.e. at about 10 or higher. Suitable amphoteric surfactants include, but are not limited to, amphocarboxylates, alkylamidoalkylamine, alkyl substituted amino acids, amphophosphates, carboxyalkyl alkyl polyamines, and mixtures thereof, wherein the alkyl group contains from about C₈ to about C₂₂ carbon atoms. Particularly suitable amphoteric surfactants include, but are not limited to, lauroamphodiacetate, lauriminodipropionate, cocoamphopropionate, lauroamphodipropionic acid, capryloamphohydroxypropylsulfonate, and mixtures thereof. The amount of amphoteric surfaclant in the compositions of the present invention may range from about 0.1% to about 20%, e.g. from about 0.5% to about 15% or from about 1.0% to about 10% by weight, based on the total weight of the composition.

The amphoteric surfactants are disclosed herein without a counter ion. One skilled in the art would readily recognize that under the pH conditions of the compositions of the present invention, the amphoteric surfactants are either electrically neutral by virtue of having balancing positive and negative charges, or they have counter ions such as, but not limited to, alkali metal, alkaline earth, or ammonium counter ions.

The ratio of the amount of the glycoside nonionic surfactant to the sum of the amounts of the amphoteric surfactant and the betaine surfactant may range from about 1 to 4 to about 1.5 to 1. The ratio of the amount of the betaine surfactant to the amount of the amphoteric surfactant may range from about 1 to 2 to about 10 to 1. The ratio of glycoside nonionic surfactant to betaine surfactant to amphoteric surfactant may range from about 1 : 1.3 : 2.66 to about 16.5 : 10 : 1, respectively.

The compositions of the present invention are substantially free of anionic surfactant. As used herein, "substantially free" of anionic surfactant means that the compositions contain less than about 1 percent, e.g. less than about 0.5 percent or less than about 0.1 percent by weight anionic surfactant, based on the total weight of the composition. The term "anionic surfactant", as used herein, means a negatively charged surfactant, and includes but is not limited to sulfates, sulfonates, isethionates, sulfosuccinates, taurates, and mixtures thereof.

Additional surfactants may be useful in the compositions of the present invention in an amount, based upon the total weight of the compositions, from about 0.5 percent to about 5 percent. One class of nonionic surfactants useful in the present invention are polyoxyethylene derivatives of polyol esters, wherein the polyoxyethylene derivative of polyol ester (1) is derived from (a) a fatty acid containing from about 8 to about 22, and preferably from about 10 to about 14 carbon atoms, and (b) a polyol selected from sorbitol, sorbitan, glucose, α-methyl glucoside, polyglucose having an average of about 1 to about 3 glucose residues per molecule, glycerine, pentaerythritol and mixtures thereof, (2) contains an average of from about 10 to about 120, and preferably about 20 to about 80 oxyethylene units; and (3) has an average of about 1 to about 3 fatty acid residues per mole of polyoxyethylene derivative of polyol ester.

Examples of preferred polyoxyethylene derivatives of polyol esters include, but are not limited to PEG-80 sorbitan laurate and Polysorbate 20. PEG-80 sorbitan laurate, which is a sorbitan monoester of laurlc acid ethoxylated with an average of about 80 moles of ethylene oxide, is available commercially from ICI Surfactants of Wilmington, Delaware under the tradename, "Atlas G-4280." Polysorbate 20, which is the laurate monoester of a mixture of sorbitol and sorbitol anhydrides condensed with approximately 20 moles of ethylene oxide, is available commercially from ICI Surfactants of Wilmington, Delaware under the tradename "Tween 20."

Optionally, the cleansing compositions of this invention may also contain, based upon the total weight of the cleansing composition, from about 0.01 percent to about 1.0 percent, e.g. from about 0.01 percent to about 0.5 percent or from about 0.01 to about 0.2 percent of at least one primary conditioning agent.

Suitable primary conditioning agents include, but are not limited to those cationic conditioning polymers having a high molecular weight ranging from about 2,000 to about 5,000,000, e.g. from about 5,000 to about 3,000,000 or from about 100,000 to about 1,000,000.

Representative classes of such suitable cationic conditioning polymers include, but are not limited to cationic polysaccharides; cationic homopolymers and copolymers derived from acrylic and or methacrylic acid; cationic cellulose resins; cationic copolymers of dimethyldiallylammonium chloride and acrylamide and/or acrylic acid; cationic homopolymers of dimethyldiallylammonium chloride; cationic polyalkylene and ethoxypolyalkylene imines; quaternized silicones and copolymers and mixtures thereof.
For example, such cationic polymers include the cationic guar gums such as guar hydroxypropyltrimonium chloride, which is commercially available from Rhodia Incorporated, under the tradename, "Jaguar C17;" quaternized hydroxy ethyl cellulose ethers such as polyquaternium 10, which are commercially available from Amerchol Corporation under the tradename, "Ucare Polymer JR 400;" copolymers of acrylamide and dimethyldiallylammonium chloride ethers, which are also known as polyquaternium 7 and are commercially available from the Mcintyre Group Ltd. under the tradename, "Mackernium 007" or from Allied Colloids under the tradename "Salcare SC10;" copolymers of vinylpyrrolidone and quaternized branched vinylpyrrolidone, which are commercially available from BASF Corporation under the tradename, "Luviquat Care;" Polyquaternium-6, which is available commercially from Allied Colloids under the tradename, "Salcare SC30," and copolymers and mixtures thereof.

In one embodiment, the primary conditioner agent comprises, consists of, or consists essentially of, based upon the overall weight of the cleansing composition, from about 0.01 percent to about 0.5 percent, e.g. from about 0.01 percent to about 0.2 percent, cationic guar derivative and from about 0.01 percent to about 0.5 percent, e.g. from about 0.01 percent to about 0.2 percent, of a homopolymer or copolymer of diallyldimethylammonium chloride.

In another embodiment, the primary conditioning agent comprises, consists of, and/or consists essentially of, based upon the total weight of the composition, from about 0.01 percent to about 5 percent, e.g. from about 0.01 percent to about 3 percent or from about 0.01 percent to about 2 percent of a cationic conditioning polymer; from about 0.1 percent to about 5 percent, e.g. from about 0.5 percent to about 3 percent, or from about 0.1 percent to about 2.5 percent of an emollient selected from the group consisting of a diester, a triester, or a mixture thereof; and from about 0.1 percent to about 10 percent, e.g. from about 0.5 percent to about 5 percent or from about 0.75 percent to about 3 percent of a monoester emollient.

Suitable diester or triester emollients may be made via the known reaction of fatty alkoxylated esters with a straight, branched or aromatic polyol or poly acid to form a diester or triester of a straight, branched or aromatic polyol or poly acid. The diester or triester reactant is comprised of two or three fatty alkoxylated moieties, respectively, having the structure set forth in formula I.: 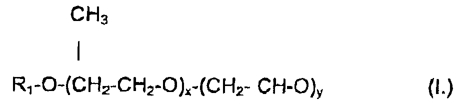 wherein:
R₁ is a saturated or unsaturated, substituted or unsubstituted, straight, branched, or aromatic fatty moiety having a carbon chain length of from about 6 to about 30 atoms; and
Each x and y are independently zero or an integer from 1 to 200, inclusive, with the proviso that the sum of x and y in each fatty alkoxylated moiety is independently between 1 and 300, inclusive, and the sum of all xs and ys in the diester or triester does not exceed 800.

The straight, branched, or aromatic polyol or polyacid is of the formula set forth in structure II.: 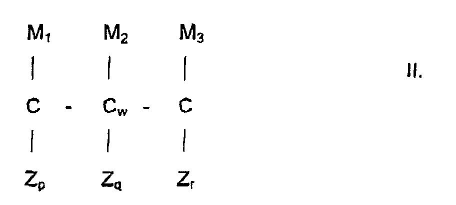 Wherein:
M₁,M₂, and M₃ are independently a hydroxy, two single bonded hydrogens, or a double bonded oxygen;
Zₚ, Z_{q} and Zᵣ are independently hydrogen or a hydroxy;
p, q, and r are independently zero or one, with the proviso that the sum of p + q + r is at least 2; and
w is zero or an integer between 1 and 20, inclusive.
One suitable diester is Di-PPG-2 Myreth-10 Adipate, which is commercially available from Croda, Incorporated under the tradename, "Cromollient SCE," and has the structure set forth in formula III.: 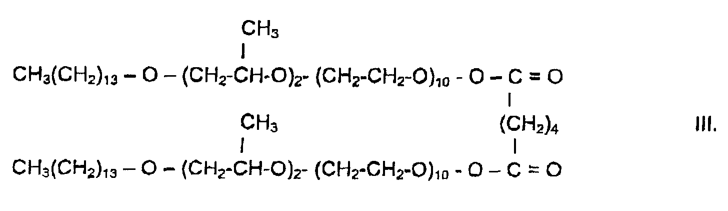 Monoester emollients suitable for use in the present invention include the esters made via the known reaction of a straight, branched or aromatic fatty acid having from about 4 carbon atoms to about 30 carbon atoms with a straight, branched or aromatic monohydric or polyhydric alcohol. If desired, the monohydric or polyhydric alcohol may be alkoxylated using known methods to improve its water solubility. The resulting monoester is of the structure set forth in formula IV.: 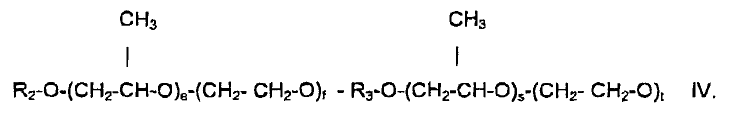 Wherein:
R₂ is a saturated or unsaturated, substituted or unsubstituted straight, branched, or aromatic fatty moiety having a carbon chain length of from about 4 to about 30 atoms;
R₃ is a saturated or unsaturated, substituted or unsubstituted, straight, branched, or aromatic monohydric or polyhydric alcohol having a carbon chain length from about 3 atoms to about 30 atoms; and

Each e, f, s, and t are independently zero or integers from 1 to 100, inclusive, with the provisos that the sum of e and f is zero or an integer between 1 and 200, inclusive, that the sum of s and t is zero or an integer between 1 and 200, inclusive, and that the sum of e, f, s, and t does not exceed 400.

Examples of monoesters suitable for use in the present invention include the glyceryl esters, such as glyceryl oleate, which is commercially available from the Goldschmidt Chemical Corporation under the tradename, "Tegin O;" PEG-7 Glyceryl Cocoate, which is commercially available from Croda Incorporated under the tradename, "Glycerox HE;" and mixtures thereof.

In one embodiment, the HLB value of one or more of the di-/tri-ester emollients and monoester emollients is less than about 11, e.g. from about 2 to about 11 or from about 4 to about 11. In another embodiment, all of the di-/tri-ester emollients and the monoester emollients have an HLB value of less than about 11, e.g. from about 2 to about 11 or from about 4 to about 11.

The cleansing composition of the present invention may optionally include a chelating agent, Examples of suitable chelating agents include those which are capable of protecting and preserving the compositions of this invention. Preferably, the chelating agent is EDTA, and more preferably is tetrasodium EDTA, available commercially from Dow Chemical Company of Midland, Michigan under the tradename, "Versene 100XL" and is present in an amount, based upon the total weight of the composition, from about 0 to about 0.5 percent, e.g. from about 0.05 percent to about 0.25 percent.

The cleansing compositions of the present invention may optionally include a preservative. Suitable preservatives include, but are not limited to, Quaternium-15, available commercially as "Dowicil 200" from the Dow Chemical Corporation of Midland, Michigan, and benzalkonium chloride and are present in the composition in an amount, based upon the total weight of the composition, from about 0 to about 0.2 percent, e.g. from about 0.05 percent to about 0.10 percent.

Optionally, a benefit agent may be included in the composition of the present invention. By "benefit agent," it is mean any active ingredient that is to be delivered into and/or onto the skin at a desired location, such as a cosmetic agent or a pharmaceutical agent. By "cosmetic agent," it is meant any ingredient that is appropriate for cosmetically treating, providing nutrients to, and/or conditioning the hair and/or skin via topical application. By "pharmaceutical agent," it is mean any drug that is either hydrophobic or hydrophilic in nature and appropriate for topical use. As used herein "medicament agents" include those agents capable of promoting recovery from injury and illness.

Examples of suitable benefit agents include, but are not limited to, depigmentation agents; reflectants; thickening agents; detangling/wet combing agents; film forming polymers; humectants; amino acid agents; antimicrobial agents; allergy inhibitors; anti-acne agents; anti-aging agents; anti-wrinkling agents, antiseptics; analgesics; antitussives; antipruritics; local anesthetics; anti-hair loss agents; hair growth promoting agents; hair growth inhibitor agents, antihistamines; antiinfectives; inflammation inhibitors; anti-emetics; anticholinergics; vasoconstrictors; vasodilators; wound healing promoters; peptides, polypeptides and proteins; deodorants and anti-perspirants; medicament agents; skin emollients and skin moisturizers; vitamins; tanning agents; skin lightening agents; antifungals such as antifungals for foot preparations; depilating agents; shaving preparations; external analgesics; perfumes; counterlrritants; hemorrhoidals; insecticides; poison ivy products; poison oak products; burn products; anti- diaper rash agents; prickly heat agents; make-up preparations; vitamins; amino acids and their derivatives; herbal extracts; retinoids; flavoids; sensates; anti-oxidants; secondary hair and/or skin conditioners; pearlescent agents; hair lighteners; chelating agents; cell turnover enhancers; coloring agents; pigments: sunscreens and the like, and mixtures thereof.

Commercially available pearlescent or opacifying agents which are capable of suspending water insoluble additives such as silicones and/or which tend to indicate to consumers that the resultant product is a conditioning shampoo are suitable for use in this invention. The pearlescent or opacifying agent is present in an amount, based upon the total weight of the composition, of from about 1 percent to about 10 percent, preferably from about 1.5 percent to about 7 percent, and more preferably, from about 2 percent to about 5 percent. Examples *of* suitable pearlescent or opacifying agents include, but are not limited to mono or diesters of (a) fatty acids having from about 16 to about 22 carbon atoms and (b) either ethylene or propylene glycol; mono or diesters of (a) fatty acids having from about 16 to about 22 carbon atoms (b) a polyalkylene glycol of the formula: HO-(JO)ₐ-H, wherein J is an alkylene group having from about 2 to about 3 carbon atoms; and a is 2 or 3; fatty alcohols containing from about 16 to about 22 carbon atoms; fatty esters of the formula: KCOOCH₂L, wherein K and L independently contain from about 15 to about 21 carbon atoms; inorganic solids insoluble in the shampoo composition, and mixtures thereof.

Examples of suitable reflectants nonexclusively include mica, alumina, calcium silicate, glycol dioleate, glycol distearate, silica, sodium magnesium fluorosilicate, and mixtures thereof.

Examples of suitable UV absorbers include benzophenone, bornelone, butyl paba, cinnamidopropyl trimethyl ammonium chloride, disodium distyrylbiphenyl disulfonate, paba, potassium methoxycinnamate, and mixtures thereof.

Commercially available thickening agents that are capable of imparting the appropriate viscosity to the compositions are suitable for use in this invention. If used, the thickener should be present in the shampoo compositions in an amount sufficient to raise the Brookfield viscosity of the composition to a value of between about 500 to about 10,000 centipoise. Examples of suitable thickening agents nonexclusively include: mono or diesters of polyethylene glycol of formula V.

HO-(CH₂CH₂O)_{z}H V.

wherein z is an integer from about 3 to about 200; fatty acids containing from about 16 to about 22 carbon atoms; fatty acid esters of ethoxylated polyols; ethoxylated derivatives of mono and diesters of fatty acids and glycerine; hydroxyalkyl cellulose; alkyl cellulose; hydroxyalkyl alkyl cellulose; and mixtures thereof. More specifically, suitable thickening agents nonexclusively include behenalkonium chloride; cetyl alcohol, quaternium 46, PG-hydroxyethyl cellulose, cocodimonium chloride, polyquaternium 6, polyquaternium 7, quaternium 18, PEG-18 glycerol oleate/cocoate, a mixture of acrylates/spirit 50 acrylate copolymer, laureth 3 and propylene glycol, which is commercially available from Goldschmidt under the tradename "Antil 208," a mixture of cocamidopropylbetaine and glyceryl laurate which is commercially available from Goldschmidt under the tradename, "Antil HS60," a mixture of propylene glycol, PEG 55, and propylene glycol oleate, which is commercially available from Goldschmidt under the tradename, "Antil 414 liquid," PEG-150 distearate which is available from the Stepan Company of Northfield, Illinois or from Comiel, S.p.A. of Bologna, Italy under the tradename, "PEG 6000 DS", and mixtures thereof.

Suitable detangling/wet combing agents nonexclusively include dioleoylamidoethyl hydroxythylmonium methosulfate, di (soyoylethyl) hydroxyethylmonium methosulfate, hydroxyethyl behenamidopropyl dimonium chloride, olealkonlum chloride, polyquaternium 47, stearalkonium chloride, tricetylmonium chloride, and mixtures thereof.

Suitable film forming polymers include those that, upon drying, produce a substantially continuous coating or film on the hair, skin, or nails. Nonexclusive examples of suitable film forming polymers include acrylamidopropyl trimonium chloride/acrylamide copolymer; corn starch/ acrylamide/ sodium acrylate copolymer; polyquaternium 10; polyquaternium 47; polyvinylmethyl/maleic anhydride copolymer; styrene/acrylates copolymers; and mixtures thereof.

Commercially available humectants which are capable of providing moisturization and conditioning propertles to the cleansing composition are suitable for use in the present invention. The humectant is preferably present in an amount of from about 0 percent to about 10 percent, more preferably from about 1.5 percent to about 7 percent, and most preferably from about 2 percent to about 5 percent, based on the overall weight of the composition. Examples of suitable humectants nonexclusively include: 1) water soluble liquid polyols selected from the group comprising glycerine, propylene glycol, hexylene glycol, butylene glycol, pentylene glycol, dipropylene glycol, and mixtures thereof; 2) polyalkylene glycol of the formula VI:

HO-(R"O)_{b}-H VI.

wherein R" is an alkylene group having from about 2 to about 4 carbon atoms
and b is an integer of from about 1 to about 10, such as PEG 4; 3)
polyethylene glycol ether of methyl glucose of formula VII:

CH₃-C₆H₁₀O₅-(OCH₂CH₂)_{c}-OH VII.

wherein c is an integer from about 5 to about 25;
4) urea; 5) fructose; 6) glucose; 7) honey; 8) lactic acid; 9) maltose; 10) sodium glucuronate; and 11) mixtures thereof.

Suitable amino acid agents include amino acids derived from the hydrolysis of various proteins as well as the salts, esters, and acyl derivatives thereof. Examples of such amino acid agents nonexclusively include amphoteric amino acids such as alkylamido alkylamines, i.e. stearyl acetyl glutamate, capryloyl silk amino acid. caprylol collagen amino acids; capryloyl kertain amino acids; capryloyl pea amino acids; cocodimonium hydroxypropyl silk amino acids; corn gluten amino acids; cysteine; glutamic acid; glycine; hair keratin amino acids; hair amino acids such as aspartic acid, threonine, serine, glutamic acid. proline, glycine, alanine, half-cystine, valine, methionine, isoleucine, leucine, tyrosine, phenylalanine, cysteic acid, lysine, histidine, arginine, cysteine, tryptophan, citrulline; lysine; silk amino acids, wheat amino acids; and mixtures thereof.

Suitable proteins include those polymers that have a long chain, i.e. at least about 10 carbon atoms, and a high molecular weight, i.e. at least about 1000, and are formed by selfcondensation of amino acids. Nonexclusive examples of such proteins include collagen, deoxyribonuclease, iodized corn protein; keratin; milk protein; protease; serum protein; silk; sweet almond protein; wheat germ protein; wheat protein; wheat protein, alpha and beta helix of keratin proteins; hair proteins, such as intermediate filament proteins, high-sulfur proteins, ultrahigh-sulfur proteins, intermediate filament-associated proteins, high-tyrosine proteins, high-glycine tyrosine proteins, tricohyalin, and mixtures thereof.

Examples of suitable vitamins nonexclusively include vitamin B complex: including thiamine, nicotinic acid, biotin, pantothenic acid, choline, riboflavin, vitamin B6. vitamin B12, pyridoxine, inositol, carnitine; vitamins A,C,D,E,K and their derivatives such as vitamin A palmitate and pro-vitamins, e.g. (i.e. panthenol (pro vitamin B5) and panthenol triacetate) and mixtures thereof.

Examples of suitable antibacterial agents nonexclusively include bacitracin, erythromycin, neomycin, tetracycline, chlortetracycline, quaternary ammonium agents such as benzethonium chloride and benzalkonium chloride, phenol, and mixtures thereof.

Examples of suitable skin emollients and skin moisturizers nonexclusively include mineral oil, lanolin, vegetable oils, isostearyl isostearate, glyceryl laurate, methyl gluceth 10, methyl gluceth 20 chitosan, and mixtures thereof.

Examples of suitable secondary hair conditioners nonexclusively volatile silicones. When used, the volatile silicone conditioning agent preferably has an atmospheric pressure boiling point less than about 220°C. The volatile silicone conditioner is present in an amount of from about 0 percent to about 3 percent, e.g. from about 0.25 percent to about 2.5 percent or from about 0.5 percent to about 1.0 percent, based on the overall weight of the composition. Examples of suitable volatile silicones nonexclusively include polydimethylsiloxane, polydimethylcyclosiloxane, hexamethyldisiloxane, cyclomethicone fluids such as polydimethylcyclosiloxane available commercially from Dow Corning Corporation of Midland, Michigan under the tradename, "DC-345" and mixtures thereof.

Examples of suitable hair moisturizers nonexclusively include panthenyl ethyl ether, phytantriol, and mixtures thereof. Examples of sunscreen agents nonexclusively include butyl methoxydibenzoylmethane, octyl methoxycinnamate, oxybenzone, octocrylene, octyl salicylate, phenylbenzimidazole sulfonic acid, ethyl hydroxypropyl aminobenzoate, menthyl anthranllate, aminobenzoic acid, cinoxate, diethanolamine methoxycinnamate, glyceryl aminobenzoate, titanium dioxide, zinc oxide, oxybenzone, padimate o. red petrolatum, and mixtures thereof,

An example of a suitable tanning agent nonexclusively includes dihydroxyacetone.

Examples of skin lightening agents nonexclusively include hydroquinone, catechol and its derivatives, ascorbic acid and its derivatives, and mixtures thereof.

Examples of suitable insecticides (including insect repellents, anti-scabies and anti-lice treatments) nonexclusively include permethrin, pyrethrin , piperonyl butoxide, imidacloprid. N,N-diethyl toluamide, which refers to the material containing predominantly the *meta* isomer, i.e., N,N-diethyl-*m*-toluamide, which is also known as DEET; compounds of the formula VIII. 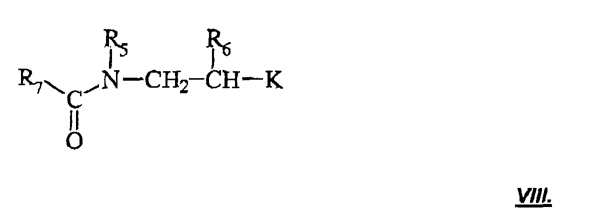 wherein
R₆ is a branched or unbranched alkyl group having about 1 to about 6 carbon atoms;
R₆ is H, methyl or ethyl;
R₇ is a branched or unbranched alkyl or alkoxy group having from about 1 to about 8 carbon atoms: and
K is a -CN or a -COOR₈ group, wherein
   R₈ is a branched or unbranched alkyl group having from about 1 to about 6 carbon atoms,
natural or synthetic pyrethroids, whereby the natural pyrethroids are contained in pyrethrum, the extract of the ground flowers of *Chrysanthemum cinerariaefolium* or *C coccineum*; and mixtures thereof. Within the structure of Formula VIII. are ethyl 3-(N-butylacetamido)propionate, wherein R₇ is a CH₃ group, R₅ is an n-butyl group, R₆ is H, K is COOR₈ and R₈ is ethyl, which is available commercially from Merck KGaA of Darmstadt, Germany under the name, "Insect Repellent 3535."

An example of an anti fungal for foot preparations nonexclusively includes tolnaftate.

Examples of suitable depilating agents nonexclusively include calcium thioglycolate, magnesium thloglycolate, potassium thioglycolate, strontium thioglycolate, and mixtures thereof.

Examples of suitable external analgesics and local anesthetics nonexclusively include benzocaine, dibucaine. benzyl alcohol, camphor, capsaicin, capsicum, capsicum oleoresin, juniper tar, menthol, methyl nicotinate, methyl salicylate, phenol, resorcinol, turpentine oil, and mixtures thereof.

Examples of suitable antiperspirants and deodorants nonexclusively include aluminium chlorohydrates, aluminium zirconium chlorohydrates, and mixtures thereof.

Examples of suitable counterirritants nonexclusively include camphor, menthol, methyl salicylate, peppermint and clove oils, ichtammol, and mixtures thereof.

An example of a suitable inflammation inhibitor nonexclusively includes hydrocortisone.

Examples of suitable hemorrhoidal products nonexclusively include the anesthetics such as benzocaine, pramoxine hydrochloride, and mixtures thereof; antiseptics such as benzethonium chloride; astringents such as zinc oxide, bismuth subgallate, balsam Peru, and mixtures thereof; skin protectants such as cod liver oil, vegetable oil, and mixtures thereof.

Examples of suitable make-up preparations nonexclusively include components for lipstick, rouge, blush, eye liner, eyeshadow powder, mascara, face powder, and mixtures thereof.

As used herein, "hair loss treatment agents" shall include agents capable of growing hair and/or agents capable of preventing the loss of hair. By "effective amount," it is meant an amount effective for treating hair loss and may range from, based upon the total weight of the cleansing composition, from about 0.001 percent to about 20 percent, e.g., preferably from about 1 percent to about 5 percent. Examples of benefit agents suitable for treating hair loss include, but are not limited to potassium channel openers or peripheral vasodilators such as minoxidil, diazoxide, and compounds such as N*-cyano-N-(tert-pentyl)-N'-3-pyridinyl-guanidine ("P-1075") as disclosed in United States Patent No.: 5,244,664, which is incorporated herein by reference; vitamins, such as vitamin E and vitamin C, and derivatives thereof such as vitamin E acetate and vitamin C palmitate; hormones, such as erythropoietin, prostaglandins, such as prostaglandin El and prostaglandin F2-alpha; fatty acids, such as oleic acid; diruretics such as spironolactone; heat shock proteins ('HSP"), such as HSP 27 and HSP 72; calcium channel blockers, such as verapamil HCL, nifedipine. and diltiazemamiloride; immunosuppressant drugs, such as cyclosporin and Fk-506; 5 alpha-reductase inhibitors such as finasteride; growth factors such as, EGF, IGF and FGF; transforming growth factor beta; tumor necrosis factor; non-steroidal anti-inflammatory agents such as benoxaprofen; retinoids such as tretinoin; cytokines, such as IL-6, IL-1 alpha, and IL-1 beta; cell adhesion molecules such as ICAM; glucorcorticoids such as betametasone; botanical extracts such as aloe, clove, ginseng, rehmannia, swertia, sweet orange, zanthoxylum, Serenoa repens (saw palmetto), Hypoxis rooperi, stinging nettle, pumpkin seeds, and rye pollen; other botanical extracts including sandlewood, red beet root, chrysanthemum, rosemary, burdock root and other hair growth promoter activators which are disclosed in DE 4330597 which is incorporated by reference in its entirety herein; homeopathic agents such as Kalium Phosphoricum D2, Azadirachta indica D2, and Joborandi DI; genes for cytokines, growth factors, and male-pattered baldness; antifungals such as ketoconazole and elubiol; antibiotics such as streptomycin; proteins inhibitors such as cycloheximide; acetazolamide; benoxaprofen; cortisone; diltiazem; hexachlorobenzene; hydantoin; nifedipine; penicillamine; phenothaiazines; pinacidil: psoralens, verapamil; zidovudine; alpha-glucosylated rutin having at least one of the following rutins: quercetin, isoquercitrin, hespeddin, naringin, and methylhesperidin, and flavonoids and transglycosidated derivatives thereof which are all disclosed in JP 7002677, which is incorporated by reference in its entirety herein; and mixtures thereof.

Examples of benefit agents suitable for use in inhibiting hair growth include: serine proteases such as trypsin; vitamins such as alpha-tocophenol (vitamin E) and derivatives thereof such as tocophenol acetate and tocophenol palmitate; antineoplastic agents, such as doxorubicin, cyclophosphamide, chlormethine, methotrexate, fluorouracil, vincristine, daunorubicin, bleomycin and hydroxycarbamlde; anticoagulants, such as heparin, heparinoids, coumaerins, detran and indandiones; antithyroid drugs, such as iodine, thiouracils and carbimazole; lithium and lithium carbonate; interferons, such as interferon alpha, interferon alpha-2a and interferon alpha-2b; retinoids, such as retinol (vitamin A), isotretinoin: glucocorticoids such as betamethasone, and dexamethosone; antihyperlipidaemic drugs, such as triparanol and clofibrate; thallium; mercury; albendazole; allopurinol; amiodarone; amphetamines; androgens; bromocriptine; butyrophenones; carbamazepine; cholestyramine; cimetidine; clofibrate; danazol; desipramine; dixyrazine; ethambutol; etionamide; fluoxetine; gentamicin, gold salts; hydantoins; ibuprofen; impramine; immunoglobulins; indandiones; indomethacin; intraconazole; levadopa; maprotiline; methysergide; metoprolol; metyrapone; nadolol; nicotinic acid; potassium thiocyanate; propranolol; pyridostimine; salicylates; sulfasalazine; terfenadine; thiamphenicol; thiouracils; trimethadione; troparanol; valproic acid; and mixtures thereof. The composition may contain, based upon the total weight of the composition, from about 0.001 percent to about 20 percent, e.g. from about 0.01 percent to about 5 percent, of a hair growth inhibiting agent.

Examples of suitable anti-aging agents include, but are not limited to inorganic sunscreens such as titanium dioxide and zinc oxide; organic sunscreens such as octyl-methyl cinnamates and derivatives thereof; retinoids; vitamins such as vitamin E, vitamin A, vitamin C, vitamin B, and derivatives thereof such as vitamin E acetate, vitamin C palmitate, and the like; antioxidants including beta carotene, alpha hydroxy acid such as glycolic acid, citric acid, lactic acid, malic acid, mandellc acid, ascorbic acid, alpha-hydroxybutyric acid, alpha-hydroxyisobutyric acid, alpha-hydroxyisocaproic acid, atrrolactic acid, alpha-hydroxyisovaleric acid, ethyl pyruvate, galacturonic acid, glucopehtonic acid, glucopheptono 1,4-lactone, gluconic acid, gluconolactone, glucuronic acid, glucurronolactone, glycolic acid, isopropyl pyruvate, methyl pyruvate, mucic acid, pyruvia acid, saccharic acid, saccaric acid 1,4-lactone, tartaric acid, and tartronic acid; beta hydroxy acids such as beta-hydroxybutyric acid, beta-phenyl-lactic acid, beta-phenylpyruvic acid; botanical extracts such as green tea, soy, milk thistle, algae, aloe, angelica, bitter orange, coffee, goldthread, grapefruit, hoellen, honeysuckle, Job's tears, lithospermum, mulberry, peony, puerarua, nice, safflower, and mixtures thereof. Suitable amounts of anti-aging agents include, based upon the total weight of the cleansing composition, from about 0.01 percent to about 10 percent, e.g. from about 0.04 percent to about 5 percent.

Examples of suitable anti-acne agents include, but are not limited to topical retinoids (tretinoin, isotretinoin, motretinide, adapalene, tazarotene, azelaic acid, retinol); salicylic acid; benzoyl peroxide; resorcinol; antibiotics such as tetracycline and isomers thereof, erythromycin, and the anti-inflammatory agents such as ibuprofen, naproxen, hetprofen; botanical extracts such as alnus, arnica, artemisia capillaris, asiasarum root, birth, calendula, chamomile, cnidium, comfrey, fennel, galla rhois, hawthrom, houttuynia, hypericum, jujube, kiwi, licorice, magnolia, olive, peppermint, philodendron, salvia, sasa albo-marginata; imidazoles such as ketoconazole and elubiol, and those described in Gollnick, H et al. 196(1) Dermatology Sebaceous Glands, Acne and Related Disorders, 119-157 (1998), which is incorporated by reference herein, and mixtures thereof. Suitable amount of anti-acne agents may be present in an amount, based upon the total weight of the cleansing composition, from about 0.01 percent to about 10 percent, e.g. from about 0.04 percent to about 5 percent.

Suitable effective amounts of depigmentation agents include, based upon the total weight of the cleansing composition, from about 0.01 percent to about 10 percent, e.g. from about 0.04 percent to about 5 percent. Examples of suitable depigmentation agents include, but are not limited to retinoids such as retinol; Kojic acid and its derivatives such as, for example, kojic dipalmitate; hydroquinone and it derivatives such as arbutin; transexamic acid; vitamins such as niacin, vitamin C and its derivatives; azelaic acid; placertia; licorice; extracts such as chamomile and green tea, and mixtures thereof.

As used herein, "dandruff treatment agent," "seborrheic dermatitis treatment agent," or a "psoriasis treatment agent," respectively, shall Include agents capable of treating the symptoms and/or the diseases of dandruff, seborrheic dermatitis, and psoriasis, respectively. By "effective amount," it is meant an amount effective for treating the disease and/or the symptoms associated therewith and preferably may range from, based upon the total weight of the cleansing composition, from about 0.001 percent to about 10 percent, e.g. from about 0.01 percent to about 5 percent. Examples of agents effective in the treatment of dandruff, seborrheic dermatitis, and psoriasis as well as the symptoms associated therewith include, but are not limited to, zinc pyrithione, shale oil and derivatives thereof such as sulfonated shale oil. selenium sulfide, sulfur; salicylic acid; coal tar; povidone-iodine, imidazoles such as ketoconazole, dichlorophenyl imidazolodioxalan, which is commercially available from Janssen Pharmaceutica, N.V., under the tradename, "Elubiol". clotrimazole, itraconazole, miconazole, climbazole, tioconazole, sulconazole, butoconazole, fluconazole, miconazolenitrite and any possible stereo isomers and derivatives thereof such as anthralin; piroctone olamine (Octopirox); selenium sulfide; ciclopirox olamine; anti-psoriasis agents such as vitamin D analogs, e.g. calcipotriol, calcitriol, and tacaleitrol; vitamin A analogs such as esters of vitamin A, e.g. vitamin A palmitate, retinoids, retinols, and retinoic acid; corticosteroids such as hydrocortisone, clobetasone, butyrate, clobetasol propionate and mixtures thereof.

Preferably the benefit agent is selected from salicylic acid, lactic acid, glycolic acid and mixtures thereof; benzethonium chloride, benzalkonium chloride, and mixtures thereof.

The amount of benefit agent to be combined with the cleansing composition may vary depending upon, for example, the resulting benefit desired and the sensitivity of the user to the benefit agent. Unless otherwise expressed herein, preferably the benefit agent is present in the cleansing composition or delivery system in an amount, based upon the total weight of the cleansing composition, from about 0.001 percent to about 20 percent, e.g. from about 0.001 percent to about 10 percent or from about 0.001 percent to about 5 percent.

The mildness of each benefit agent will depend upon the benefit agent selected and the amount of benefit agent employed. Therefore, in general, in embodiments wherein mildness is of particular concern, the composition may omit the incorporation of a benefit agent or alternatively use low amounts thereof. Similarly, the effect of the benefit agent on foam agent will depend upon the benefit agent selected and the amount of benefit agent employed. Therefore, in general, in embodiments wherein high foaming is of particular concern, the composition may omit the incorporation of a benefit agent or alternatively use low amounts thereof.

The pH of the compositions of this invention will depend upon, for example, the benefit agent selected and the type of product, e.g. shampoo, wash, etc., desired. However, the pH is typcially maintained in the range of from about 3 to about 7.5, e.g. from about 5.5 to about 7.0. For shampoo compositions, a pH of from about 5.5 to about 7.5 is typically used. Compositions of this invention having a pH from about 3 to about 5 are useful for delivering many skin care benefit agents, which include but are not limited to alpha and beta hydroxy acids such as salicylic acid, glycolic acid, lactic acid, and mixtures thereof, to the skin. Compositions having a pH of about 7 or higher are useful for delivering quatemary antibacterial agents such as benzalkonium chloride and benzethonium chloride to the skin.

The above described cleansing composition may be prepared by combining the desired components in a suitable container and mixing them under ambient conditions in any conventional mixing means well known in the art, such as via mechanically stirred propeller, paddle, and the like. Although the order of mixing is not critical, it is preferable to pre-blend certain components, such as the fragrance and the nonionic surfactant before adding such components into the main mixture.

The compositions of the present invention are preferably used in shampoos, washes, baths, gels, lotions, creams, and the like for cleansing purposes. In one embodiment, the composition may be incorporated into a bath fizz ball, such as those described in U.S. Patent Nos. 4,650,661; 4,666,707; and 4,002,758; which are incorporated herein by reference.

The composition of the present invention may be used on the body in conjunction with any personal cleansing implement known in the art such as a wipe, a washcloth, a mesh or apertured film, pouf, sponge, brush and the like. In one embodiment, the composition may be marketed together with one or more of such implements in a kit. The implements may be loaded with appropriate skin care compositions and packaged as a wet implement, such as wet wipes. The composition of the present invention may be loaded onto the implement by dipping the implement in the composition, by spraying the composition onto the implement, and other means known in the art.

Alternatively, the wet implement may be dried through the use of heating equipment, vacuum driers, or other means known in the art to provide a dry implement, such as dry wipes. Alternatively, the composition of the present invention may be applied in the form of a concentrate to the implement in order to provide a dry article. These dry articles may then be sold dry with the intent that the consumer will consequently wet the article with water when ready for use.

The compositions of the present invention have a low degree of ocular irritancy. By "low degree of ocular irritancy" it is meant that the composition is not more irritating to eyes than pure sterile water.

Several examples are set forth below to further illustrate the nature of the invention and the manner of carrying it out. However, the invention should not be considered as being limited to the details thereof.

### Examples

The following test method is used in the below Examples:
Human Ocular Irritancy Test: This is a double-blinded, randomized, two (2) cell study, with test materials being applied once. The test material, which is a 10% dilution of a test sample having a temperature of 37-38°C, is instilled in one eye and sterile purified water is installed in the other eye of each panelist. Ten (10) panelists are required to complete the study.

Following the randomized order of testing the panelists and of assigning which material to instill and into which eye that material shall be instilled, one (1) drop of the appropriate test material is instilled into the first eye of the panelist followed by instillation of the sterile water into the second eye. A new sterile disposable eyedropper Is used for each test material and disposed of after being used on only one panelist's eye. All instillations are performed either by an investigator or by a trained technician. For each eye, an independent tissue is used to blot any lacrimation that falls from that respective eye. Within 30 seconds, or as closely as possible following instillation, panelists are asked to grade stinging sensation to their eyes utilizing the following criteria: 0 = within normal limits;1 = mild, very slight, 2 = moderate, and 3 = severe. At ¼ hour and 1 hour post-instillation, panelists are again asked to grade stinging sensation to eyes.

### Example 1: Preparation of Cleansing Compositions

Three independent sample formulations were prepared utilizing the materials listed in Table 1:

**Table 1**

| CTFA NAME | #1* | #2* | #3* | #4* |
|---|---|---|---|---|
| Decyl Glucoside | 3.0 | 3.0 | 3.0 | 3.6 |
| Cocamidopropylbetaine | 3.0 | 6.0 | - | 4.7 |
| Disodium Lauroamphodiacetate | 3.0 | - | 6.0 | 0.7 |

| | | | | |
|---|---|---|---|---|
| * expressed in terms of weight percentage | | | | |

Each of the samples was independently prepared by adding the appropriate parts of active surfactant to water such that the resultant quantity equals 100 parts. Each solution was then stirred until a homogenous, clear solution was obtained.

Foam stability evaluation was accomplished using an Airspray® mini-foamer 0.4cc pump. The pump is capable of extruding a constant volume of liquid and allowing it to mix with air, which therefore creates enough energy to yield a foam. With time, the foam eventually breaks down. However, a dense, stable foam will retain its foam structure for longer periods of time than a loose, unstable foam. The procedure for using this foam test was as follows:
1) Each sample was inserted into an independent mini foamer, which was then pumped with 1 stroke onto a porous substrate that allowed for wicking and draining of the liquid.
2) An initial, visual foam assessment was made on the pumped sample, based upon a scale of 1-10. A rating of "10" indicated a very dense, creamy, high quality foam while a "1" indicated a fully drained, foam remnant.
3) Periodic assessments were made of the pumped sample while the foam gradually degraded.
The results are reported in Table 2.

**Table 2**

| Time (minutes) | #1 | #2 | #3 | #4 |
|---|---|---|---|---|
| 1 | 10 | 10 | 10 | 10 |
| 2 | 10 | 9 | 9 | 10 |
| 3 | 10 | 7 | 7 | 9 |
| 4 | 10 | 3 | 2 | 9 |
| 5 | 8 | 1 | 1 | 7 |
| 10 | 8 | 1 | 1 | 7 |
| 14 | 6 | 1 | 1 | 4 |
| 15 | 4 | 1 | 1 | 2 |

As can be seen from the data above, the compositions of the present invention provided a long lasting stable foam that was dense, creamy, and of high quality. In particular, as shown in Samples 1 and 4, the stable foam retained its approximate initial foam height and quality, i.e. stayed white/pastel in color, was very dense, and did not degrade, for a time longer than about 3 minutes, and such stable foam lasted over twice as long as that produced by comparative cleansers.

### Example 2: Preparation of Cleansing Composition with Conditioner

A moisturizing cleansing composition was prepared according to the materials and amounts listed in Table 3 as follows:

**Table 3**

| Tradename / Supplier | INCI Name | %w/w |
|---|---|---|
| Plantaren 2000 from Cognis Corporation | Decyl Glucoside | 6.0 |
| Lamesoft P065 from Cognis Corporation | | 3.00 |
| | Water | 1.05 |
| | Coco glucoside | 1.05 |
| | Glyceryl oleate | 0.9 |
| PEG 6000 DS from Stepan Company | PEG 150 distearate | 1.4 |
| Atlas G4280 from Uniqema | POE 80 sorbitan laurate | 5.0 |
| Water | Deionized water | 59.67 |
| Polymer JR from Amerchol Corporation | Polyquaternium 10 | 0.12 |
| Glucquat 125 from Amerchol Corporation | Lauryl methyl gluceth-10/hydroxypropyldi-monium chloride | 1.00 |
| Tegobetaine L-7 (30%) from Goldschmidt Chemical Corporation | Cocamidopropyl betalne | 12.00 |
| Monateric 949J from Uniqema | Disodium lauroamphodiacetate | 2.00 |
| Polyox WSR-205 from Amerchol | PEG 14-M | 0.10 |

| Corporation | | |
|---|---|---|
| Glycerin from Cognis Corporation | Glycerin | 0.50 |
| Cromollient SCE from Croda Inc. | Di-PPG-Myreth-10-Adipate | 1.00 |
| Lipovol J from Lipo Chemicals, Inc. | Jojoba oil | 0.1 |
| Drakeol 7 from Penreco | Mineral oil | 0.10 |
| Fragrance | Fragrance | 0.50 |
| Dowicil 200 from Dow Chemical Company | Quaternium 15 | 0.05 |
| Versene 100XL from Dow Chemical Company | Tetrasodium EDTA | 0.46 |
| Euperlan PK 3000 from Cognis Corporation | | 2.00 |
| | Cocamidopropyl betaine | |
| | Glycol distearate | |
| | Laureth-4 | |
| Citric acid (20%) | Citric acid | As needed |

The following Pre-Mixtures were prepared:
Pre-Mixture 1: PEG-14M and glycerin were mixed in an independent container under ambient conditions.
Pre-Mixture 2: Di-PPG-2-myreth-10-adipate, mineral oil, and jojoba oil were mixed in an independent container under ambient conditions.
Pre Mixture 3: The fragrance and 1 part of POE-80 sorbitan laurate were mixed in an independent container under ambient conditions.
Component amounts in this procedure are given in terms of parts by weight of active to prepare 100 parts of the cleansing composition.

The decyl glucoside, cocoglucoside, and glyceryl oleate were mixed in an independent container under ambient conditions until the resulting solution was clear. The solution was then heated to a temperature of about 50 °C to 55 °C with constant stirring. PEG 150 distearate was added thereto with stirring at constant temperature until the PEG 150 distearate was dissolved therein. As the resulting solution was cooled to 40 °C, the following components were added thereto sequentially with stirring, and the solution was homogeneous before the addition of each subsequent component: (4 parts) POE-80 sorbitan laurate; (59.67 parts) water; polyquaternium-10; lauryl methyl gluceth-10 hydroxypropyldimonium chloride: cocamidopropylbetaine; disodium lauroamphodiacetate; PEG-14M/glycerin pre-mix; and Di-PPG-2-myreth-10-adipate/mineral oil/jojoba oil pre-mix.

After the resulting mixture was cooled to a temperature of about 40 °C. the following components were added thereto sequentially, and the solution was homogeneous before the addition of each subsequent component: fragrance/POE-80 sorbitan laurate premixture, quaternium-15, tetrasodium EDTA, glycol distearate, and laureth - 4. After sodium chloride was added thereto, the pH of the resulting solution was adjusted with citric acid to about 6.8, The remaining water was added thereto with stirring until the final solution was homogeneous. The viscosity of the resulting solution was 1300 cps as measured by a Brookfield DV-I⁺ Viscometer using a #2 spindle and speed of 6 rpm.

### Example 3: Ocular Irritancy Test

The human ocular sting test was performed utilizing the composition produced in accordance with Example 2. The results of the study are shown in Table 4.

**Table 4**

| Sample | Stinging To Eye Score |
|---|---|
| Composition of Example 2 | 0 |
| sterile purified water | 0 |

This Example shows that the compositions of this invention were found to be no more stinging to the eyes than sterile purified water.

## Claims

1. A cleansing composition
comprising:
at least one glycoside nonionic surfactant;
at least one betaine surfactant; and
at least one amphoteric surfactant, wherein the composition is substantially free of anionic surfactants.

2. The composition of claim 1 wherein the composition possesses a low degree of ocular irritancy and yields a long-lasting stable foam.

3. The composition according to claim 1 wherein the glycoside nonionic surfactant is selected from coco glucoside, octyl glucoside, decyl glucoside, lauryl glucoside, and mixtures thereof.

4. The composition according to claim 3 wherein the amount of glycoside nonionic surfactant ranges from about 1% to about 10% by weight, based on the total weight of the composition.

5. The composition according to claim 1 wherein the glycoside nonionic surfactant is decyl glucoside and ranges from about 1% to about 10% by weight, based on the total weight of the composition; the betaine surfactant is cocamidopropylbetaine and ranges from about 1% to about 10% by weight, based on the total weight of the composition; and the amphoteric surfactant is lauroamphodiacetate and ranges from about 1% to about 10% by weight, based on the total weight of the composition.

6. The composition according to claim 1 wherein the ratio of the glycoside nonionic surfactant to the sum of the amounts of the amphoteric surfactant and the betaine surfactant ranges from about 1 to 4 to about 1.5 to 1.

7. The composition of any preceding claim further comprising from about 0.01 percent to about 5 percent of a cationic conditioning polymer; from about 0.1 percent to about 5 percent of an emollient selected from the group consisting of a diester, a triester, or a mixture thereof; and from about 0.1 percent to about 10 percent of a monoester emollient.

8. The composition of any preceding claim further comprising a benefit agent.

9. The use of the composition of any one of claims 1-8 with an implement selected from the group consisting of a wipe, a washcloth, a mesh or apertured film, pouf, sponge, and a brush.

10. A kit comprised of the composition of any one of claims 1-8 and an implement selected from the group consisting of a wipe, a washcloth, a mesh or apertured film, pouf, sponge, and a brush.
